# EUROPÄISCHE PATENTSCHRIFT

(11) **EP 3 458 237 B1**
(45) Veröffentlichungstag und Bekanntmachung des Hinweises auf die Patenterteilung: **28.02.2024**
(21) Anmeldenummer: 17731069.5
(22) Anmeldetag: 22.05.2017
(51) Int. Cl.: B25J 15/08, C12M 1/00, G01N 35/00, B25J 9/04, B25J 15/02

(54) **GREIFER**
GRIPPER
PRÉHENSEUR

(30) Priorität: 20.05.2016 DE 102016109317
(43) Veröffentlichungstag der Anmeldung: 27.03.2019
(73) Patentinhaber: Andreas Hettich GmbH & Co. KG, 78532 Tuttlingen (DE)
(72) Erfinder: EBERLE, Klaus-Günter, 78532 Tuttlingen (DE)
(74) Vertreter: Puschmann Borchert Kaiser Klettner Patentanwälte Partnerschaft mbB
(86) Internationale Anmeldenummer: PCT/EP2017/062275
(87) Internationale Veröffentlichungsnummer: WO 2017/198875

(56) Entgegenhaltungen:
- JP-A- H04 240 080
- JP-A- 2000 326 276
- US-A- 5 700 046
- US-A1- 2012 251 275

## Beschreibung

Die Erfindung betrifft eine Greifvorrichtung, insbesondere zum Ergreifen, translatorischen Verfahren und Drehen eines Laborgefäßes gemäß der im Oberbegriff des Patentanspruches 1 angegeben Art.

In der Labortechnik werden zum Lagern und zur Analyse von Proben, Mikroorganismen, Zellkulturen oder Ähnlichem verschiedene Laborgefäße verwendet. Besonders häufig kommen Petrischalen zum Einsatz. Petrischalen sind in verschiedenen Größen erhältlich und gut stapel- und lagerbar.

Petrischalen werden oft in Stapeln so gelagert, dass der Deckel sich unten befindet und der Behälter oben, wobei die offene Seite des Behälters nach unten weist. Diese Ausrichtung hat unter anderem den Vorteil, dass so eine Ansammlung von Kondenswasser auf dem Nährboden im Behälter vermieden werden kann. Zur Analyse oder Behandlung der enthaltenen Proben werden Petrischalen oft stapelweise in ein Magazin einer entsprechenden Verarbeitungsvorrichtung eingesetzt und dann innerhalb der Vorrichtung einzeln zur Analyse- bzw. Behandlungseinheit verfahren, wo sie schließlich zur Verarbeitung so gedreht werden, dass die offene Seite des Behälters nach oben weist.

Zum Verfahren der Petrischalen innerhalb der Verarbeitungsvorrichtung ist gewöhnlich eine Greifvorrichtung vorgesehen, mit der die Petrischalen ergriffen werden und sowohl translatorisch verfahren als auch gedreht werden können. Dabei weist die Greifvorrichtung einen Lagerkörper und einen in dem Lagerkörper drehbar gelagerten Greifer auf, der einen Drehkörper und in dem Drehkörper angeordnete Greiffinger umfasst. Von den Greiffingern ist zumindest einer schwenkbar ausgebildet. Der Drehkörper wirkt mit einem Drehantrieb für die Drehbewegung gegenüber dem Lagerkörper zusammen, und der Lagerkörper wirkt mit einem Verfahrantrieb zum translatorischen Verfahren des Lagerkörpers mit dem Drehkörper gegenüber der Trageinheit zusammen. Ferner ist ein Fingerantrieb vorgesehen zum Verschwenken oder zum Verfahren zumindest eines Greiffingers gegenüber dem Drehkörper aus einem Öffnungsstellung in eine Schließstellung und vice versa. Der Greiffingerantrieb wirkt mit den Greiffingern zusammen. Ein solcher Antrieb ist beispielsweise aus der US 2014/0377038 A1 bekannt.

In der Praxis erweist es sich häufig als Problem, dass herkömmliche Greifvorrichtungen wegen des recht hohen Gewichts und der Vielzahl der sich bewegenden Teile schnell verschleißen. Zudem sind sie im Falle des Verschleißes oder einer Beschädigung nur schwer auszutauschen. Des Weiteren wirken sich die hohen bewegten Massen nachteilig auf die Genauigkeit der Bewegung der einzelnen Teile der Greifvorrichtung aus.

Ein Greifarm für Handhabungsgeräte ist aus der DE 35 04 233 A1 bekannt. Hier wird das Problem dadurch gelöst, dass der Antriebsmotor greiferfern angeordnet ist und der Antrieb der Greiffinger über ein entsprechendes Getriebe erfolgt. Eine ähnliche Lösung ist auch aus der EP 0 995 555 A1 bekannt.

Die DE 102 47 731 A offenbart einen Greifautomaten, dessen Greifwerkzeug mit einem Antrieb kuppelbar ist.

Die Druckschrift JP H04 240080 A, JP 2000326276 A und US 5 700 046 A offenbaren jeweils einen Greifer, der permanent mit dem Antrieb verbunden ist. Bei der JP H04 240080 A ist auch der Lagerkörper nur zusammen mit der Trageinheit translatorisch verfahrbar. Bei der JP 2000326276 A ist ein Drehen nicht möglich.

Die gattungsbildende Druckschrift der Erfindung bildet die US 2012/0251275 A1, welche eine Greifvorrichtung zum Ergreifen, translatorischen Verfahren und Drehen eines Laborgefäßes für Proben, Mikroorganismen, Zellkulturen oder Ähnlichem aufweist. Die Greifvorrichtung ist mit einer Trageinheit, einem Lagerkörper und einem in dem Lagerkörper drehbar gelagerten Greifer versehen. Der Greifer weist einen Drehkörper auf, in dem Greiffinger angeordnet sind. Von den Greiffingern sind beide schwenkbar im Drehkörper gelagert. Der Drehkörper wirkt mit einem Drehantrieb für die Drehbewegung gegenüber dem Lagerkörper zusammen. Der Lagerkörper ist in der Trageinheit translatorisch verfahrbar gelagert und wirkt mit einem Verfahrantrieb zum translatorischen Verfahren des Lagerkörpers mit dem Drehkörper gegenüber der Trageinheit zusammen. Die Greiffinger wirken mit einem Greiffingerantrieb zum Verschwenken der Greiffingers gegenüber dem Drehkörper aus einer Öffnungsstellung in eine Schließstellung und vice versa zusammen.

Aufgabe der Erfindung ist es, unter Vermeidung der genannten Nachteile eine Greifvorrichtung zu schaffen, die präzise Bewegungen ausführt, insbesondere langlebig ist und bei Bedarf schnell und einfach auszutauschen ist.

Diese Aufgabe wird durch die kennzeichnenden Merkmale des Patentanspruches 1 in Verbindung mit seinen Oberbegriffsmerkmalen gelöst.

Die Unteransprüche bilden vorteilhafte Weiterbildungen der Erfindung.

Der Erfindung liegt die Erkenntnis zugrunde, dass die Präzision der Greifvorrichtung dadurch erhöht werden kann, dass die für die Ausrichtung entscheidenden Teile, nämlich der Greifer, möglichst leicht ausgeführt wird und dies dadurch erreicht werden kann, dass ein Teil des Greiferantriebs außerhalb des Greifers angeordnet ist und somit im Betrieb am Ausgangspunkt verbleibt und vom weiteren Teil des Greiferantriebs entkoppelt ist, während der Greifer nach dem Ergreifen mit dem Laborgefäß verfährt und dieses dreht. Zudem ist der im Verschleißfalle vorzunehmende Austausch einfacher.

Die Erfindung betrifft eine Greifvorrichtung, insbesondere zum Ergreifen, translatorischen Verfahren und Drehen eines Laborgefäßes für Proben, Mikroorganismen, Zellkulturen oder Ähnlichem. Die Greifvorrichtung weist eine Trageinheit, einen Lagerkörper und einen in dem Lagerkörper drehbar gelagerten Greifer auf, der einen Drehkörper und Greiffinger umfasst. In dem Drehkörper sind die Greiffinger angeordnet, von denen zumindest einer schwenkbar im Drehkörper gelagert ist. Der Drehkörper wirkt dabei mit einem Drehantrieb für die Drehbewegung gegenüber dem Lagerkörper zusammen. Der Lagerkörper ist in der Trageinheit translatorisch verfahrbar gelagert und wirkt mit einem Verfahrantrieb zum translatorischen Verfahren des Lagerkörpers mit dem Drehkörper gegenüber der Trageinheit zusammen. Die Greiffinger wirken mit einem Greiffingerantrieb zum Verschwenken zumindest eines Greiffingers gegenüber dem Drehkörper aus einer Öffnungsstellung in eine Schließstellung und vice versa zusammen. Erfindungsgemäß ist zumindest ein erster Teil des Greiffingerantriebs zum Verschwenken der Greiffinger außerhalb des Drehkörpers angeordnet. Ein zweiter Teil des Greiffingerantriebs ist im Drehkörper angeordnet, wobei zum Ergreifen des Laborgefäßes der erste Teil des Greiffingerantriebs und der zweite Teil des Greiffingerantriebs miteinander gekoppelt werden, ansonsten die beiden Teile des Greiffingerantriebs voneinander getrennt sind. Eine Aufnahme des zweiten Teils des Greiffingerantriebs ist vorgesehen, so dass ein Betätigungselement des ersten Teils des Greiffingerantriebs bei entsprechender translatorischer Ausrichtung der Aufnahme des Drehkörpers zum Betätigungselement zumindest einen Greiffinger durch Bewegen in die Aufnahme auf ein sich anschließende Getriebe öffnen kann. Der Greifer wird dadurch leichter und weniger komplex im Aufbau und kann daher mit einfacheren Mitteln exakter bewegt und geführt werden. So wird der Verschleiß verringert, wodurch die Kosten für die Greifvorrichtung deutlich geringer werden. Der Greifer wird dadurch im Hinblick auf die Bauhöhe kompakter und kann so besser in eine Messeinheit eingeführt werden, die ggf. empfindlich für Fremdlicht ist.

Bevorzugt werden die Greiffinger durch Bewegen des Betätigungselements geöffnet. Die Greiffinger sind durch Federn in ihre Schließstellung vorgespannt. Die Federkraft der Federn wirkt auf die Greiffinger mit Zurückfahren des Betätigungselements. Die Greiffinger bewegen sich dann in eine Schließstellung und bei Vorhandensein eines Laborgefäßes in eine Greifstellung.

Vorzugsweise ist zumindest der erste Teil des Greiffingerantriebs in der Trageinheit angeordnet. Dadurch können die Maße der Greifvorrichtung kompakt gehalten werden, der Greifer wird leichter und ein exakteres Bewegen ist mit einfacheren Mitteln möglich. Dies erweitert den Einsatzbereich der Greifvorrichtung. Die Trageinheit wird nicht bewegt. Insofern verringern sich in der Gesamtvorrichtung die im Betrieb bewegten Massen.

Gemäß einer Ausführungsform der Erfindung wird der erste Teil des Greiffingerantriebs gebildet. Hierdurch werden die schweren Teile des Antriebs von den bewegten Teilen auf einfache Weise entfernt und die bewegten Massen reduzieren sich im Betrieb.

Um ein exaktes Bewegen zu ermöglichen, umfasst der erste Teil einen Elektromotor, insbesondere einen Schrittmotor.

Vor allem kann der Elektromotor mit dem dem Getriebe zugeordneten Betätigungselement zusammenwirken, wobei der Elektromotor über das Betätigungselement das Getriebe antreibt und darüber zumindest einen Greiffinger bewegt. Das Betätigungselement und ein entsprechender Angriffsbereich für das Betätigungselement des Getriebes bilden eine Kupplungseinrichtung, so dass das erste Teil und das zweite Teil bedarfsweise in der vorbestimmten Position (Greifstellung) miteinander gekuppelt werden können. Die Länge und die Formgebung des Betätigungselements können leicht an die baulichen Gegebenheiten angepasst werden. Durch den Schrittmotor können die Greiffinger präzise zum Ergreifen von Petrischalen unterschiedlicher Größen gesteuert werden. Die Greifvorrichtung wird dadurch sicherer und flexibler einsetzbar.

Insbesondere ist zwischen Betätigungselement und Elektromotor ein die Drehbewegung des Elektromotors in eine translatorische Bewegung umsetzendes Getriebe zwischengeschaltet. Hierdurch sind eine kompaktere Bauweise und ein genaues, zielgerichtetes Bewegen des Greifers ohne weiteres möglich.

Gemäß einem weiteren Aspekt der Erfindung umfasst der Lagerkörper den Antrieb für die Drehbewegung des Drehkörpers. Hierdurch werden Gewicht und Maße des Greifers umfassend den Drehkörper und die Greiffinger, also die bewegten Massen, weiter reduziert, wodurch der Verschleiß des Greifers weiter verringert wird. Dies senkt die Betriebskosten der Greifvorrichtung. Zudem werden die Voraussetzungen für exakte, zielgerichtete Bewegungen des Greifers in einem engen Toleranzbereich geschaffen.

Es ist weiterhin zweckmäßig, dass zwei Greiffinger vorgesehen sind, wobei beide Greiffinger schwenkbar oder translatorisch verschiebbar im Drehkörper gelagert sind und mit dem Getriebe des zweiten Teils des Greiffingerantriebs so verbunden sind, dass eine synchrone, gegenläufige Bewegung beider Greiffinger beim Öffnen und Schließen erfolgt. Auf diese Weise wird sichergestellt, dass das Laborgefäß zentriert ergriffen wird. Ist der Greifer und das Laborgefäß zueinander exakt ausgerichtet, kommt es zu keiner translatorischen Bewegung beim Ergreifen des Laborgefäßes mittels der Greiffinger des Greifers.

Vorzugsweise umfasst der Greiffinger einen Antriebsabschnitt, der mit dem Getriebe des Antriebs zusammenwirkt. Der Antriebsabschnitt des Greiffingers kann dabei auf der dem freien Ende entfernt gelegenen Seite angeordnet sein und einen Verzahnungsbereich aufweisen, der mit einem Zahnrad des Getriebeteils zusammenwirkt. Zahnradverbindungen dieser Art können zum einen sehr genau angesteuert werden, was die Präzision der Greifvorrichtung verbessert. Zum anderen ist der Verschleiß gering, was wiederum die Sicherheit verbessert und Betriebskosten senkt.

Insbesondere ist der Greiffinger um eine Schwenkachse schwenkbar gelagert. Der Greiffinger erstreckt sich im Wesentlichen in einer Greiferebene, welche parallel zur Schwenkebene des Greiffingers ausgerichtet ist. Die Schwenkachse kann dabei im Wesentlichen zwischen einem Fingerabschnitt und dem Antriebsabschnitt angeordnet sein und der Antriebsabschnitt vollständig im Drehgehäuse angeordnet sein. Hierdurch ergibt sich eine kompakte Bauweise und ein einfacherer Antrieb für die Bewegung der Greiffinger.

Nach einer Ausführungsform der Erfindung weist jeder Greiffinger einen Antriebsabschnitt auf, sind die Verzahnungsbereiche der Greiffinger einander gegenüberliegend angeordnet und greift ein Zahnrad des Getriebeteils in den jeweiligen Verzahnungsbereich der Greiffinger ein. Auf einfache Weise wird dadurch eine gleichförmige, synchrone gegenläufige Bewegung der Greiffinger gewährleistet, welche in Abhängigkeit der Fertigungsgüte der Zahnverbindung mit einer hohen Präzision ausgeführt werden kann.

Bei einer bevorzugten Ausführungsform sind die Greiffinger im Bereich des zu ergreifenden Laborgefäßes dem Verlauf des Außenbereiches des Laborgefäßes angepasst. Dies verringert die Gefahr, dass das Laborgefäß beim Verfahren aus dem Greifer rutscht. Die Sicherheit der Greifvorrichtung wird weiter erhöht.

Bei einer vorteilhaften Weiterbildung der Erfindung sind an den Greiffingern in Richtung des zu ergreifenden Laborgefäßes gerichtete Haltefinger vorgesehen, welche in einem vorbestimmten Abstand und einer vorbestimmten Ausrichtung zueinander angeordnet sind. Dadurch wird das Laborgefäß, beispielsweise eine Petrischale, zusätzlich zum von den Greiffingern auf ihren Außenumfang ausgeübten Druck auch durch Lager festgelegt, an denen die Petrischale lagert. Dies ist eine Sicherung gegen ein unbeabsichtigtes Lösen des Laborgefäßes während des Betriebs der Greifvorrichtung.

Sehr günstig ist es weiterhin, wenn in einer Position, in der die Haltefinger das Laborgefäß halten (Halteposition), jedem Haltefinger des einen Greiffingers ein Haltefinger des anderen Greiffingers diametral gegenüberliegend so angeordnet ist, dass die Längsachsen dieser beiden Haltefinger auf einer Achse liegen. Dies verbessert die Sicherheit dahingehend, dass auch ein schief, also nicht parallel zur Ebene des Greifers, eingesetztes Laborgefäß nicht aus der Greifvorrichtung rutschen kann.

Gemäß einer Ausführungsform der Erfindung hat es sich als vorteilhaft erwiesen, dass ein Greiffinger jeweils zwei Haltefinger aufweist. In der Halteposition bilden die Längsachsen von zwei gegenüberliegenden Haltefingern jeweils eine Achse und nehmen die beiden Achsen zueinander einen Winkel von 90° ein. Diese Anordnung der Haltefinger bietet die größtmögliche Sicherheit gegen ein Herausrutschen der Petrischale aus der Greifvorrichtung.

Vorzugsweise weisen die Haltefinger eine Vielzahl von Außenflächen auf, welche zur Greiferebene in einem stumpfen Winkel ausgerichtet sind. Dadurch werden Reflexionen von Umgebungslicht vermieden, die insbesondere bei der Analyse des Inhalts des Laborgefäßes störenden Einfluss haben können. Um diese Reflexionen noch weiter zu verringern, können die Haltefinger schwarz bzw. matt und dunkel gefärbt sein.

In einer alternativen Ausführungsform weist der Antrieb des Lagerkörpers eine in der Trageinheit gelagerte Schienenführung auf, auf welcher der Lagerkörper translatorisch verfahrbar ist. Diese Schienenführung gewährleistet ein sicheres und beliebig oft exakt wiederholbares Verfahren des Greifers und verbessert dadurch die Präzision und Lebensdauer der Greifvorrichtung insgesamt.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine dreidimensionale Ansicht einer Greifvorrichtung nach der Erfindung von schräg oben vorne mit einer ergriffenen Petrischale;
- Fig. 2: eine dreidimensionale Ansicht der Greifvorrichtung wie in Fig. 1 mit der ergriffenen und um 180° gedrehten Petrischale;
- Fig. 3: eine dreidimensionale Detailansicht der Greifvorrichtung von schräg oben hinten mit einem Liftantrieb zum vertikalen Verfahren der Petrischale in der Position, in der ein Behälter der Petrischale ergriffen wird, wobei der Behälter in dieser Ansicht noch nicht ergriffen ist;
- Fig. 4: eine dreidimensionale Detailansicht der Greifvorrichtung wie in Fig. 3 bei dem der Liftantrieb in eine Position nach unten verfahren ist und der Behälter der Petrischale ergriffen ist.
- Fig. 5: eine dreidimensionale Ansicht der Greifvorrichtung wie in Fig. 4, jedoch in der translatorisch verfahrenen Position ohne Liftantrieb mit gedrehter Petrischale im Bereich des Analysegeräts, und
- Fig. 6: eine dreidimensionale Ansicht der Greifvorrichtung nach der Erfindung von schräg oben hinten vor dem translatorischen Verfahren in den Bereich eines Analysegeräts.

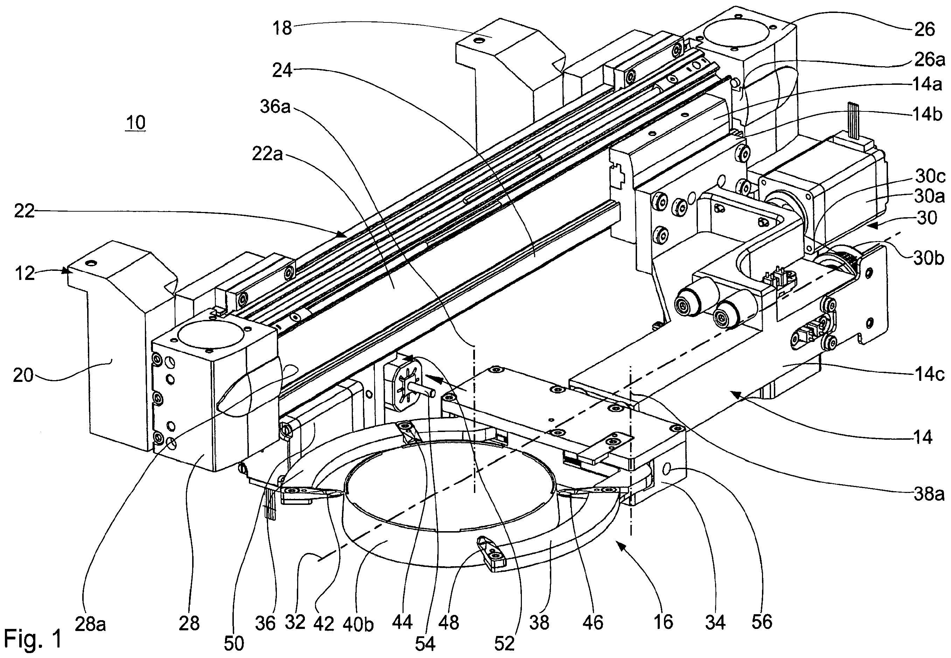

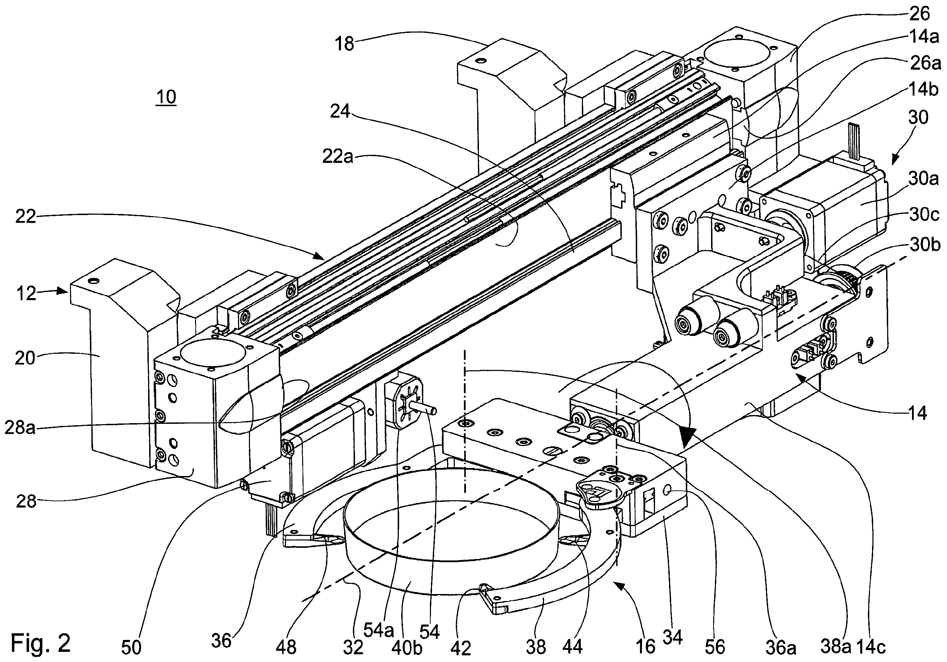

In der Figur 1 ist in einer dreidimensionalen Ansicht eine Greifvorrichtung 10 dargestellt, welche eine Trageinheit 12, einen Lagerkörper 14 und einen Greifer 16 umfasst. In Figur 2 ist eine entsprechende Darstellung gezeigt, jedoch sind der Greifer 16 und der Lagerkörper 14 um 180° gedreht.

Die Trageinheit 12 weist zwei Auflager 18 und 20 auf, welche mit einem hier nicht dargestellten Gehäuse oder einem Tragegestell verbindbar sind. Zwischen den Auflagern 18, 20 erstreckt sich eine Schiene 22, auf der der Lagerkörper 14 translatorisch verfahrbar gelagert ist. Hierfür greift ein Fuß 14a des Lagerkörpers 14 formschlüssig in eine Halteschiene 24, welche parallel zur Schiene 22 verläuft und auf einer Seitenfläche 22a der Schiene 22 angeordnet ist. Mit Bezug auf Figur 1 oberhalb der Halteschiene 24 erstreckt sich die Seitenfläche 22a, welche ebenfalls eine Anlage- und Gleitfläche für den Lagerkörper 14 bildet. An der Seitenfläche 22a liegt ein Teil des Lagerkörpers 14 an und gleitet beim translatorischen Verfahren auf dieser Seitenfläche 22a entlang.

An den Enden der Schiene 22 sind Antriebsblöcke 26, 28 vorgesehen, in welchen ein Motor als auch Umlenkrollen für ein Antriebsseil - alternativ einen Antriebsriemen - gelagert sind. Das Antriebsseil ist mit seinen Enden jeweils mit dem Fuß 14a des Lagerkörpers 14 verbunden, verläuft in die Antriebsblöcke 26, 28 über die dort gelagerten Umlenkrollen aus den Antriebsblöcken 26, 28 wieder heraus innerhalb der Schiene 22. Die Lagerblöcke 26, 28 sind jeweils mit Anschlägen 26a, 28a versehen, welche dem Fuß 14a des Lagerkörpers 14 zugeordnet sind. Aus Gründen der Übersicht ist das Antriebsseil nicht dargestellt. Die Umlenkrollen und der Motor, welcher eine zugeordnete Umlenkrolle antreibt, befinden sich innerhalb des Lagerblockes 26 und eine weitere Umlenkrolle innerhalb des Lagerblockes 28. Diese Umlenkrolle ist nicht angetrieben, sondern nur drehbar gelagert. Die Umlenkrollen und der Motor sind in den Figuren nicht sichtbar.

Auf den Fuß 14a ist eine Basisplatte 14b aufgeschraubt, auf der wiederum ein Winkelkörper 14c angeordnet ist. Die Basisplatte 14b und der Winkelkörper 14c können aber auch einstückig als durchgehendes Teil ausgebildet sein. Der Winkelkörper 14c erstreckt sich zunächst senkrecht zur Basisplatte 14b von der Schiene 22 weg und anschließend parallel zur Trageinheit 12. Im Hinblick auf die Figur 1 und 2 erstreckt sich der Winkelkörper 14c parallel zur Richtung von Antriebsblock 26 zu Antriebsblock 28. An dem freien Ende des Winkelkörpers 14c greift der Greifer 16 in den Winkelkörper 14c ein, ist dort drehbar gelagert und mit einem Drehantrieb 30 verbunden. Der Drehantrieb 30 besteht aus einem elektrischen Antriebsmotor 30a und einem Drehgetriebe, welche am dem Greifer 16 entfernt gelegenen Ende des Winkelkörpers 14c angeordnet sind. Das Drehgetriebe umfasst eine konzentrisch zur Drehachse 32 des Greifers 16 und mit dem Greifer 16 verbundene Antriebsrolle 30b, welche über einen nur angedeuteten Antriebsriemen 30c mit einer Motorwelle des Antriebsmotors 30a verbunden ist. Über den Drehantrieb 30 wird der Greifer 16 bedarfsweise um die Drehachse 32 gedreht, vorzugsweise um 180°.

Der Greifer 16 besteht aus dem in den Winkelkörper 14c eingreifenden Drehkörper 34, der im Wesentlichen quaderförmig ausgebildet ist. In dem Drehkörper 34 sind zwei Greiffinger 36 und 38 und ein Getriebe 34a gelagert, wobei die Greiffinger 36 und 38 jeweils um eine Schwenkachse 36a, 38a synchron gegenläufig schwenkbar sind. Alternativ sind diese translatorisch verfahrbar im Drehkörper 34 gelagert. Die Schwenkachsen 36a und 38a erstrecken sich senkrecht zu einer Greiferebene, welche durch die Greiffinger 36 und 38 aufgespannt wird. Die Schwenkachsen 36a und 38a teilen den jeweiligen Greiffinger 36 bzw. 38 in einen Fingerabschnitt sowie einen Antriebsabschnitt, der einen Verzahnungsbereich aufweist und mit einem Zahnrad oder Zahnstange des Getriebes 34a innerhalb des Drehkörpers 32 zusammenwirkt. Der Fingerabschnitt ist außerhalb des Drehkörpers 32 angeordnet, ist an die Form des zu ergreifenden Laborgefäßes in Form einer Petrischale 40 bogenförmig angepasst und weist jeweils zwei Haltefinger 42, 44 auf der einen Seite und 46 und 48 auf der anderen Seite auf.

Die Haltefinger 42 und 46 sowie 44 und 48 sind diametral gegenüberliegend angeordnet. Die Haltefinger 42 bis 48 sind so ausgerichtet und angeordnet, dass die Längsachsen der Haltefinger 42 und 46 auf der einen Seite und die Längsachsen der Haltefinger 44 und 48 auf der anderen Seite identisch sind. Die Längsachse der Haltefinger 42 und 46 und die Längsachse der Haltefinger 44 und 48 sind zueinander um 90° versetzt ausgerichtet. Alternativ können die Finger auch parallel zueinander ausgerichtet sein.

Unterhalb der Schiene 22 ist ein elektrischer Schrittmotor 50 angeordnet, der mit einem Getriebe 52 zusammenwirkt. Das Getriebe 52 ist wirktechnisch mit einem Betätigungselement 54 in Form einer Stange verbunden. Über den Schrittmotor 50 und das Getriebe 52 wird das Betätigungselement 54 vorbestimmt nach außen gefahren und wieder zurückgefahren. Der Motor 50, das Getriebe und das Betätigungselement 54 bilden den ersten Teil des Antriebs für die Greiffinger 36, 38. Dem Betätigungselement 54 ist eine Aufnahme 56 im Lagerkörper 14 des Greifers 16 zugeordnet, welcher als Eingang und Führung für das Betätigungselement 54 dient. An die Aufnahme 56 schließt sich das hier nicht näher dargestellte Getriebe 34a an, welches die translatorische Bewegung des Betätigungselementes 54 in gegenläufige Schwenkbewegungen der Greiffinger 36, 38 umwandelt. Dieses im Drehkörper 34 angeordnete Getriebe 34a besteht im Wesentlichen aus einer durch das Betätigungselement 54 angetriebenen Zahnstange, einem in die Zahnstange eingreifenden Zahnrad, das mit dem Antriebsabschnitt des jeweiligen Greiffingers 36 bzw. 38 zusammenwirkt. Der Antriebsabschnitt ist im Wesentlichen durch einen Verzahnungsbereich gebildet, der in das Zahnrad eingreift und so ausgeformt ist, dass mit Drehen des Zahnrades jeweils die Schwenkbewegung des Greiffingers 36 und 38 erfolgt.

Eine Aufnahme 56 ist auf beiden Seiten des Lagerkörpers 34 vorgesehen, so dass unabhängig von der Drehlage des Greifers 16 das Betätigungselement 54 bei entsprechender translatorischer Ausrichtung der Aufnahme 56 des Lagerkörpers 34 zum Betätigungselement 54 die Greiffinger 36, 38 durch Bewegen in die Aufnahme 56 auf das sich anschließende Getriebe zu öffnen kann. Alternativ kann die Aufnahme 56 auch auf einer Seite des Lagerkörpers 34 vorgesehen sein. Die Greiffinger 36, 38 sind durch Federn in ihre Schließstellung vorgespannt. Mit Zurückfahren des Betätigungselementes 54 wirkt die Federkraft der Federn auf die Greiffinger 36, 38 und diese bewegen sich dann in eine Schließstellung und bei Vorhandensein einer Petrischale 40 in eine Halteposition.

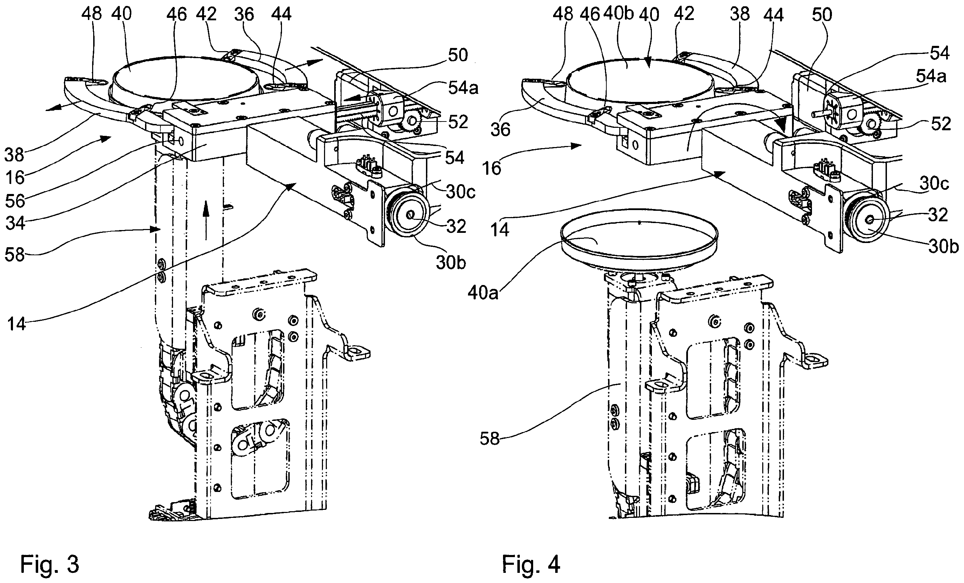

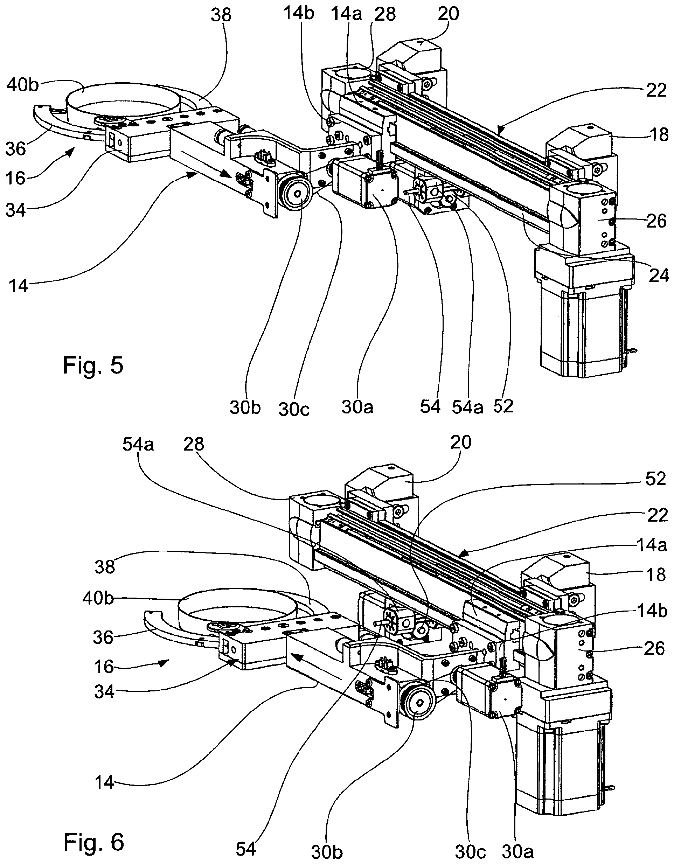

Die Petrischale 40 besteht aus einem Behälter 40b und einem Deckel 40a, siehe Figur 4. Figur 1 zeigt den von den Greiffingern 36, 38 ergriffenen Behälter 40b, der nach unten offen ist. In Figur 2 ist der Behälter 40b um 180° gedreht. In Figur 3 ist die Petrischale 40 aus Behälter 40b und Deckel 40a noch geschlossen, wobei auch hier der Behälter 40b oben ist und der Deckel 40a unten. Unterhalb der Petrischale 40 ist eine Liftvorrichtung 58 vorgesehen, welche die Petrischale 40 vertikal aus einer Aufnahmeposition in eine Position verfährt, in der der Behälter 40b durch den Greifer 16 ergriffen werden kann. Diese Position ist in Figur 3 dargestellt. Bevor die Petrischale 40 durch die Liftvorrichtung 58 in diese Position verfährt, verfährt das Betätigungselement 54 in die Aufnahme 56, betätigt das im Lagerkörper 34 angeordnete Getriebe und öffnet dadurch die Greiffinger 36, 38. Somit werden der erste und der zweite Teil des Greiffingerantriebs miteinander gekoppelt. Die Liftvorrichtung 58 verfährt nun die Petrischale 40 nach oben in eine vorbestimmte Position, in der der Behälter 40b der Petrischale 40 von den Greiffingern 36, 38 über die Haltefinger 42 bis 48 ergriffen wird. Hierfür verfährt das Betätigungselement 54 in seine Ausgangsposition zurück, über die Federkraft schließen sich die Greiffinger 36, 38 und der Behälter 40b der Petrischale 40 wird ergriffen. Der erste und der zweite Teil des Greiffingerantriebs werden dadurch wieder voneinander entkoppelt. Anschließend fährt die Liftvorrichtung 58 mit dem Deckel 40a so weit nach unten, dass der Greifer 16 um 180° gedreht werden kann. Diese Position ist in den Figuren 2 und 5 dargestellt. Die beiden Teile des Greiffingerantriebs sind somit nur in der vorbestimmten Position miteinander gekoppelt, und insbesondere auch hier nur zum Ergreifen der Petrischale 40. Ansonsten sind die beiden Teile wieder voneinander getrennt.

Anschließend verfährt der Lagerkörper 14 zusammen mit dem Greifer 16 und dem Behälter 40b der Petrischale 40 translatorisch in eine Position unterhalb eines Analysegerätes, siehe Figur 5 das jedoch aus Gründen der Übersicht nicht dargestellt ist. In dieser Position wird die in dem Behälter 40b enthaltene Kultur über bildgebende Verfahren analysiert. Anschließend verfährt der Lagerkörper 14 mit dem Greifer 16 wieder zurück, der Greifer 16 dreht um 180° den Behälter 40b, die Liftvorrichtung 58 mit dem Deckel 40a der Petrischale 40 verfährt nach oben bis der Behälter 40b in den Deckel eingreift. Der Greifer 16 befindet sich nunmehr wieder in der vorbestimmten Position und der erste Teil und der zweite Teil des Greiffingerantriebs werden miteinander gekoppelt, indem das Betätigungselement 54, welches durch eine Führung 54a geführt wird, wodurch die Rotationsbewegung des Schrittmotors 50 durch das Getriebe 54b in eine translatorische Bewegung des Betätigungselementes 54 umgesetzt wird, wieder in die Aufnahme 56 einfährt, und dadurch die Greiffinger 36, 38 öffnet, dadurch die Petrischale 40 freigibt und die Liftvorrichtung 58 die Petrischale 40 zum weiteren Transport nach unten verfährt.

Die Haltefinger 42 bis 48 weisen eine Vielzahl von zueinander im Winkel stehenden einzelnen Außenflächen auf, welche zur Greiferebene in einem stumpfen Winkel ausgerichtet sind. Hierdurch wird eine sogenannte "Stealth"-Form geschaffen, die ermöglicht, dass es keine störenden Reflexionen bei der bildverarbeitenden Analyse gibt. Hierbei sind die Finger zudem auch schwarz und in dunkler Farbe gehalten.

Die Erfindung zeichnet sich dadurch aus, dass der erste Teil des Greiffingerantriebs zum Öffnen der Greiffinger 36, 38 außerhalb der bewegten Teile angeordnet ist und nur für den Greifvorgang in der vorbestimmten Position mit dem zweiten Teil des Greiffingerantriebs gekoppelt wird. Hierdurch werden die Voraussetzungen geschaffen, dass die bewegten Massen der Greifvorrichtung im Betrieb geringer sind und ein exakteres Greifen und Verfahren ermöglicht wird. Zudem wird das System im Hinblick auf die Bauhöhe sehr kompakt.

### Bezugszeichenliste

- 10: Greifvorrichtung
- 12: Trageinheit
- 14: Lagerkörper
- 14a: Fuß
- 14b: Basisplatte
- 14c: Winkelkörper
- 16: Greifer
- 18: Auflager
- 20: Auflager
- 22: Schiene
- 22a: Seitenfläche
- 24: Halteschiene
- 26: Antriebsblock
- 26a: Anschlag
- 28: Antriebsblock
- 28a: Anschlag
- 30: Drehantrieb
- 30a: Antriebsmotor
- 30b: Antriebsrolle
- 30c: Antriebsriemen
- 32: Drehachse
- 34: Drehkörper
- 34a: Getriebe - zweiter Teil des Greiffingerantriebs
- 36: Greiffinger
- 36a: Schwenkachse
- 38: Greiffinger
- 38a: Schwenkachse
- 40: Petrischale
- 40a: Deckel der Petrischale
- 40b: Behälter der Petrischale
- 42: Haltefinger
- 44: Haltefinger
- 46: Haltefinger
- 48: Haltefinger
- 50: Schrittmotor
- 52: Getriebe des Antriebs des Betätigungselements 54
- 54: Betätigungselement
- 54a: Führung des Betätigungselementes
- 56: Aufnahme im Drehkörper 34 des Greifers 16
- 58: Liftvorrichtung

## Patentansprüche

1. Greifvorrichtung (10), insbesondere zum Ergreifen, translatorischen Verfahren und Drehen eines Laborgefäßes (40) für Proben, Mikroorganismen, Zellkulturen oder Ähnlichem, mit einer Trageinheit (12), einem Lagerkörper (14) und einem in dem Lagerkörper (14) drehbar gelagerten Greifer (16), der einen Drehkörper (34) aufweist, in dem Greiffinger (36, 38) angeordnet sind, von denen zumindest einer schwenkbar im Drehkörper (34) gelagert ist, wobei der Drehkörper (34) mit einem Drehantrieb (30) für die Drehbewegung gegenüber dem Lagerkörper (14) zusammenwirkt, der Lagerkörper (14) in der Trageinheit (12) translatorisch verfahrbar gelagert ist und mit einem Verfahrantrieb zum translatorischen Verfahren des Lagerkörpers (14) mit dem Drehkörper (34) gegenüber der Trageinheit (12) zusammenwirkt, und die Greiffinger (36, 38) mit einem Greiffingerantrieb (50, 52, 54) zum Verschwenken zumindest eines Greiffingers (36, 38) gegenüber dem Drehkörper (34) aus einer Öffnungsstellung in eine Greifstellung und vice versa zusammenwirken, **dadurch gekennzeichnet, dass** zumindest ein erster Teil des Greiffingerantriebs (50, 52, 54) zum Verschwenken der Greiffinger (36, 38) außerhalb des Drehkörpers (34) und ein zweiter Teil des Greiffingerantriebs (34a) im Drehkörper (34) angeordnet ist, wobei nur zum Ergreifen des Laborgefäßes (40) der erste Teil des Greiffingerantriebs (50, 52, 54) und der zweite Teil des Greiffingerantriebs (52) miteinander gekoppelt werden, ansonsten die beiden Teile des Greiffingerantriebs (50, 52, 54) voneinander getrennt sind, worin eine Aufnahme (56) des zweiten Teils des Greiffingerantriebs (50, 52, 54) vorgesehen ist, so dass ein Betätigungselement (54) des ersten Teils des Greiffingerantriebs (50, 52, 54) bei entsprechender translatorischer Ausrichtung der Aufnahme (56) des Drehkörpers (34) zum Betätigungselement (54) zumindest einen Greiffinger (36, 38) durch Bewegen in die Aufnahme (56) auf ein sich anschließendes Getriebe (34a) öffnen kann.

2. Greifvorrichtung nach Anspruch 1, **dadurch gekennzeichnet, dass** alle Greiffinger (36, 38) durch Bewegen des Betätigungselements (54) geöffnet werden, und dabei die Greiffinger (36, 38) durch Federn in ihre Schließstellung vorgespannt sind, und mit Zurückfahren des Betätigungselements (54), die Federkraft der Federn auf die Greiffinger (36, 38) wirkt und diese sich dann in eine Schließstellung und bei Vorhandensein eines Laborgefäßes (40) in eine Greifstellung bewegen.

3. Greifvorrichtung nach Anspruch 1 oder 2, **dadurch gekennzeichnet, dass** zumindest der erste Teil des Greiffingerantriebs (50, 52, 54) in der Trageinheit (12) angeordnet ist.

4. Greifvorrichtung nach einem der vorangehenden Ansprüche, **dadurch gekennzeichnet, dass** der erste Teil (50, 52, 54) durch einen motorischen Antrieb (50, 52, 54) gebildet ist, insbesondere umfasst der erste Teil (50, 52, 54) einen Elektromotor, insbesondere Schrittmotor (50).

5. Greifvorrichtung nach Anspruch 4, **dadurch gekennzeichnet, dass** der Elektromotor (50) mit einem dem Getriebe (34a) zugeordneten Betätigungselement (54) zusammenwirkt, wobei der Elektromotor (50) über das Betätigungselement (54) das Getriebe (34a) antreibt und zumindest einen Greiffinger (36, 38) bewegt, vorzugsweise ist zwischen Betätigungselement (54) und Elektromotor (50) eine die Drehbewegung des Elektromotors (50) in eine translatorische Bewegung umsetzendes Getriebe (52) zwischengeschaltet.

6. Greifvorrichtung nach einem der vorangehenden Ansprüche, **dadurch gekennzeichnet, dass** der Lagerkörper (14) den Antrieb (30) für die Drehbewegung des Drehkörpers (34) umfasst.

7. Greifvorrichtung nach einem der vorangehenden Ansprüche 3 bis 6, **dadurch gekennzeichnet, dass** zwei Greiffinger (36, 38) vorgesehen sind, wobei insbesondere beide Greiffinger (36, 38) schwenkbar oder translatorisch verfahrbar im Drehkörper (34) gelagert sind und mit dem Getriebe (34a) so verbunden sind, dass eine synchrone, gegenläufige Bewegung beider Greiffinger (36, 38) beim Öffnen und Schließen erfolgt.

8. Greifvorrichtung nach einem der vorangehenden Ansprüche 3 bis 7, **dadurch gekennzeichnet, dass** der Greiffinger (36, 38) einen Antriebsabschnitt umfasst, der mit dem Getriebe (34a) des Antriebs zusammenwirkt, insbesondere ist der Antriebsabschnitt des Greiffingers (36, 38) auf der dem freien Ende entfernt gelegenen Seite angeordnet und weist einen Verzahnungsbereich auf, der mit einem Zahnrad des Getriebes (34a) zusammenwirkt.

9. Greifvorrichtung nach Anspruch 8, **dadurch gekennzeichnet, dass** der Greiffinger (36, 38) um eine Schwenkachse (36a, 38a) schwenkbar gelagert ist, der Greiffinger (36, 38) sich im Wesentlichen in einer Greiferebene erstreckt, welche parallel zur Schwenkebene des Greiffingers (36, 38) ausgerichtet ist, vorzugsweise ist dabei die Schwenkachse (36a, 38a) im Wesentlichen zwischen einem Fingerabschnitt und dem Antriebsabschnitt angeordnet ist.

10. Greifvorrichtung nach Anspruch 9, **dadurch gekennzeichnet, dass** der Antriebsabschnitt vollständig im Drehgehäuse (34) angeordnet ist.

11. Greifvorrichtung nach einem der Ansprüche 7 bis 10, **dadurch gekennzeichnet, dass** jeder Greiffinger (36, 38) einen Antriebsabschnitt aufweist, die Verzahnungsbereiche der Greiffinger (36, 38) einander gegenüberliegend angeordnet sind und ein Zahnrad des Getriebes (34a) in den jeweiligen Verzahnungsbereich der Greiffinger (36, 38) eingreift.

12. Greifvorrichtung nach einem der vorangehenden Ansprüche, **dadurch gekennzeichnet, dass** die Greiffinger (36, 38) im Bereich des zu ergreifenden Laborgefäßes (40) dem Verlauf des Außenbereiches des Laborgefäßes (40) angepasst sind.

13. Greifvorrichtung nach einem der vorangehenden Ansprüche, **dadurch gekennzeichnet, dass** an den Greiffingern (36, 38) in Richtung des zu ergreifenden Laborgefäßes (40) gerichtete Haltefinger (42 bis 48) vorgesehen sind, welche in einem vorbestimmten Abstand und einer vorbestimmten Ausrichtung zueinander angeordnet sind, insbesondere ist in der Greiferstellung jedem Haltefinger (42 bis 48) des einen Greiffingers (36, 38) ein Haltefinger (42 bis 48) des anderen Greiffingers (36, 38) diametral gegenüberliegend so angeordnet, dass die Längsachse dieser beiden Haltefinger (42 bis 48) auf einer Achse liegen und die Haltefinger (42 bis 48) weisen dabei eine Vielzahl von Außenflächen auf, welche zur Greiferebene in einem stumpfen Winkel ausgerichtet sind.

14. Greifvorrichtung nach einem der vorangehenden Ansprüche, **dadurch gekennzeichnet, dass** der Antrieb des Lagerkörpers (14) eine in der Trageinheit (12) gelagerte Schienenführung (22, 24) aufweist, auf welcher der Lagerkörper (14) translatorisch verfahrbar ist.

## Claims

1. Gripping device (10), in particular for gripping, translationally moving, and rotating a laboratory vessel (40) for samples, microorganisms, cell cultures, or the like, comprising a support unit (12), a bearing body (14), and a gripper (16) that is rotatably mounted in the bearing body (14), which gripper (16) has a rotating body (34), in which gripper fingers (36, 38) are arranged, at least one of which is pivotally mounted in the rotating body (34), which rotating body (34) interacts with a rotary drive (30) for rotational movement relative to the bearing body (14), which bearing body (14) is mounted in the support unit (12) in a translationally movable manner and interacts with a moving drive for translationally moving the bearing body (14) with the rotating body (34) with respect to the support unit (12), and the gripper fingers (36, 38) interact with a gripper finger drive (50, 52, 54) for pivoting at least one gripper finger (36, 38) relative to the rotating body (34) from an open position into a gripping position, and vice versa, **characterized in that** at least one first part of the gripper finger drive (50, 52, 54) for pivoting the gripper fingers (36, 38) is arranged outside the rotating body (34), and a second part of the gripper finger drive (34a) is arranged in the rotating body (34), with the first part of the gripper finger drive (50, 52, 54) being connectable to the second part of the gripper finger drive (52) only for the purpose of gripping the laboratory vessel (40), otherwise the two parts of the gripper finger drive (50, 52, 54) are separated from one another, wherein a receptacle (56) of the second part of the gripper finger drive (50, 52, 54) is provided so that, when the receptacle (56) of the rotating body (34) is correspondingly translationally aligned with an actuating element (54), the actuating element (54) of the first part of the gripper finger drive (50, 52, 54) can open at least one gripper finger (36, 38) by moving it into the receptacle (56) onto an adjoining gear unit (34a).

2. Gripping device according to claim 1, **characterized in that** all gripper fingers (36, 38) are opened by moving the actuating element (54), and in this process, the gripper fingers (36, 38) are pretensioned in their closed position by springs, and, when the actuating element (54) is retracted, the spring force of the springs acts on the gripper fingers (36, 38), causing the latter to move into a closed position thereof, and, if a laboratory vessel (40) is present, into a gripping position thereof.

3. Gripping device according to claim 1 or 2, **characterized in that** at least the first part of the gripper finger drive (50, 52, 54) is arranged in the support unit (12).

4. Gripping device according to any one of the preceding claims, **characterized in that** the first part (50, 52, 54) is constituted by a motor drive (50, 52, 54), in particular the first part (50, 52, 54) comprises an electric motor, in particular a stepper motor (50).

5. Gripping device according to claim 4, **characterized in that** the electric motor (50) interacts with an actuating element (54) associated with the gear unit (34a), with the electric motor (50) driving the gear unit (34a) via the actuating element (54) and moving at least one gripper finger (36, 38), wherein a gear unit (52) that converts the rotary movement of the electric motor (50) into a translational movement is preferably interposed between the actuating element (54) and the electric motor (50).

6. Gripping device according to any one of the preceding claims, **characterized in that** the bearing body (14) comprises the drive (30) for rotary movement of the rotating body (34).

7. Gripping device according to any one of claims 3 to 6 above, **characterized in that** two gripper fingers (36, 38) are provided, wherein in particular both gripper fingers (36, 38) are pivotally or translationally movably mounted in the rotating body (34) and are connected to the gear unit (34a) in such a way that a synchronous, counter-rotating movement of both gripper fingers (36, 38) takes place during opening and closing.

8. Gripping device according to any one of claims 3 to 7 above, **characterized in that** the gripper finger (36, 38) comprises a drive portion which interacts with the gearbox (34a) of the drive, in particular the drive portion of the gripper finger (36, 38) is arranged on the side remote from the free end and has a toothed area that meshes with a gearwheel of the gearbox (34a).

9. Gripping device according to claim 8, **characterized in that** the gripper finger (36, 38) is pivotally mounted about a pivot axis (36a, 38a), with the gripper finger (36, 38) extending substantially in a gripper plane which is aligned parallel to the pivot plane of the gripper finger (36, 38), with the pivot axis (36a, 38a) preferably being arranged substantially between a finger portion and the drive portion.

10. Gripping device according to claim 9, **characterized in that** the drive portion is arranged completely within the rotating housing (34).

11. Gripping device according to any one of claims 7 to 10 above, **characterized in that** each gripper finger (36, 38) has a drive portion, the toothed areas of the gripper fingers (36, 38) are arranged opposite one another, and a gearwheel of the gearbox (34a) engages in the respective toothed area of the gripper fingers (36, 38).

12. Gripping device according to any one of the preceding claims, **characterized in that,** in the area of the laboratory vessel (40) to be gripped, the gripper fingers (36, 38) are adapted to the contours of the outer area of the laboratory vessel (40).

13. Gripping device according to any one of the preceding claims, **characterized in that** the gripper fingers (36, 38) are provided with holding fingers (42 to 48) that are oriented in the direction of the laboratory vessel (40) to be gripped, which holding fingers are arranged at a predetermined distance from, and in a predetermined orientation relative to, one another, in particular, in the gripping position, a holding finger (42 to 48) of one gripper finger (36, 38) is arranged diametrically opposite to each holding finger (42 to 48) of the other gripper finger (36, 38) in such a way that the longitudinal axes of these two holding fingers (42 to 48) are located on one axis, and the holding fingers (42 to 48) have a plurality of outer surfaces which extend at an obtuse angle to the gripper plane.

14. Gripping device according to any one of the preceding claims, **characterized in that** the drive of the bearing body (14) has a rail guide (22, 24) which is mounted in the support unit (12) and on which the bearing body (14) can be translationally moved.

## Revendications

1. Dispositif de préhension (10), en particulier pour la saisie, le déplacement en translation et la rotation d'un récipient de laboratoire (40) pour des échantillons, des micro-organismes, des cultures cellulaires ou similaires, avec une unité porteuse (12), un corps de support (14) et un élément de préhension (16) monté en rotation dans le corps de support (14), qui présente un corps rotatif (34), dans lequel sont disposés des doigts de préhension (36, 38), dont au moins l'un est monté de manière pivotante dans le corps rotatif (34), dans lequel le corps rotatif (34) coopère avec un entraînement rotatif (30) pour le mouvement de rotation par rapport au corps de support (14), le corps de support (14) est monté de manière à pouvoir être déplacé en translation dans l'unité porteuse (12) et coopère avec un entraînement de déplacement pour le déplacement en translation du corps de support (14) avec le corps rotatif (34) par rapport à l'unité porteuse (12), et les doigts de préhension (36, 38) coopèrent avec un entraînement de doigt de préhension (50, 52, 54) pour le pivotement d'au moins un doigt de préhension (36, 38) par rapport au corps rotatif (34) à partir d'une position d'ouverture dans une position de préhension et vice versa, **caractérisé en ce qu'**au moins une première partie de l'entraînement de doigt de préhension (50, 52, 54) pour le pivotement des doigts de préhension (36, 38) à l'extérieur du corps rotatif (34) et une deuxième partie de l'entraînement de doigt de préhension (34a) est disposée dans le corps rotatif (34), dans lequel la première partie de l'entraînement de doigt de préhension (50, 52, 54) et la deuxième partie de l'entraînement de doigt de préhension (52) sont accouplées l'une à l'autre uniquement pour la préhension du récipient de laboratoire (40), autrement les deux parties de l'entraînement de doigt de préhension (50, 52, 54) sont séparées l'une de l'autre, dans lequel un logement (56) de la deuxième partie de l'entraînement de doigt de préhension (50, 52, 54) est prévu, de sorte qu'un élément d'actionnement (54) de la première partie de l'entraînement de doigt de préhension (50, 52, 54) lors d'une orientation en translation correspondante du logement (56) du corps rotatif (34) par rapport à l'élément d'actionnement (54) peut ouvrir au moins un doigt de préhension (36, 38) par mouvement dans le logement (56) sur un engrenage (34a) se raccordant.

2. Dispositif de préhension selon la revendication 1, **caractérisé en ce que** tous les doigts de préhension (36, 38) sont ouverts par mouvement de l'élément d'actionnement (54), et ce faisant les doigts de préhension (36, 38) sont précontraints par des ressorts dans leur position de fermeture, et avec retour de l'élément d'actionnement (54), la force de ressort des ressorts agit sur les doigts de préhension (36, 38) et ceux-ci se meuvent alors dans une position de fermeture et en présence d'un récipient de laboratoire (40) dans une position de préhension.

3. Dispositif de préhension selon la revendication 1 ou 2, **caractérisé en ce qu'**au moins la première partie de l'entraînement de doigt de préhension (50, 52, 54) est disposée dans l'unité porteuse (12).

4. Dispositif de préhension selon l'une quelconque des revendications précédentes, **caractérisé en ce que** la première partie (50, 52, 54) est formée par un entraînement moteur (50, 52, 54), en particulier la première partie (50, 52, 54) comprend un moteur électrique, en particulier moteur pas à pas (50).

5. Dispositif de préhension selon la revendication 4, **caractérisé en ce que** le moteur électrique (50) coopère avec un élément d'actionnement (54) associé à l'engrenage (34a), dans lequel le moteur électrique (50) par l'intermédiaire de l'élément d'actionnement (54) entraîne l'engrenage (34a) et fait se mouvoir au moins un doigt de préhension (36, 38), de préférence un engrenage (52) convertissant le mouvement de rotation du moteur électrique (50) en un mouvement de translation est interposée entre l'élément d'actionnement (54) et le moteur électrique (50).

6. Dispositif de préhension selon l'une quelconque des revendications précédentes, **caractérisé en ce que** le corps de support (14) comprend l'entraînement (30) pour le mouvement de rotation du corps rotatif (34).

7. Dispositif de préhension selon l'une quelconque des revendications précédentes 3 à 6, **caractérisé en ce que** deux doigts de préhension (36, 38) sont prévus, dans lequel en particulier les deux doigts de préhension (36, 38) sont montés de manière pivotante ou de manière à pouvoir être déplacés en translation dans le corps rotatif (34) et sont reliés à l'engrenage (34a), de sorte qu'un mouvement synchrone opposé des deux doigts de préhension (36, 38) s'effectue lors de l'ouverture et de la fermeture.

8. Dispositif de préhension selon l'une quelconque des revendications précédentes 3 à 7, **caractérisé en ce que** le doigt de préhension (36, 38) comprend une section d'entraînement, qui coopère avec l'engrenage (34a) de l'entraînement, en particulier la section d'entraînement du doigt de préhension (36, 38) est disposée sur le côté éloigné de l'extrémité libre et présente une zone de denture, qui coopère avec une roue dentée de l'engrenage (34a).

9. Dispositif de préhension selon la revendication 8, **caractérisé en ce que** le doigt de préhension (36, 38) est monté de manière à pouvoir pivoter autour d'un axe de pivotement (36a, 38a), le doigt de préhension (36, 38) s'étend sensiblement dans un plan d'élément de préhension, lequel est orienté parallèlement au plan de pivotement du doigt de préhension (36, 38), de préférence l'axe de pivotement (36a, 38a) est disposé ce faisant sensiblement entre une section de doigt et la section d'entraînement.

10. Dispositif de préhension selon la revendication 9, **caractérisé en ce que** la section d'entraînement est disposée entièrement dans le boîtier rotatif (34).

11. Dispositif de préhension selon l'une quelconque des revendications 7 à 10, **caractérisé en ce que** chaque doigt de préhension (36, 38) présente une section d'entraînement, les zones de denture des doigts de préhension (36, 38) sont disposées à l'opposé les unes des autres et une roue dentée de l'engrenage (34a) s'insère dans la zone de denture respective des doigts de préhension (36, 38).

12. Dispositif de préhension selon l'une quelconque des revendications précédentes, **caractérisé en ce que** les doigts de préhension (36, 38) dans la zone du récipient de laboratoire (40) à saisir sont adaptés au tracé de la zone extérieure du récipient de laboratoire (40).

13. Dispositif de préhension selon l'une quelconque des revendications précédentes, **caractérisé en ce que** des doigts de retenue (42 à 48) dirigés dans la direction du récipient de laboratoire (40) à saisir sont prévus sur les doigts de préhension (36, 38), lesquels sont disposés à une distance prédéfinie et dans une orientation prédéfinie les uns par rapport aux autres, en particulier, un doigt de retenue (42 à 48) de l'autre doigt de préhension (36, 38) est disposé de manière diamétralement opposée à chaque doigt de retenue (42 à 48) du un doigt de préhension (36, 38) dans la position d'élément de préhension, de sorte que l'axe longitudinal de ces deux doigts de retenue (42 à 48) se situe sur un axe et les doigts de retenue (42 à 48) présentent ce faisant une pluralité de surfaces extérieures, lesquelles sont orientées par rapport au plan d'élément de préhension selon un angle obtus.

14. Dispositif de préhension selon l'une quelconque des revendications précédentes, **caractérisé en ce que** l'entraînement du corps de support (14) présente un guidage sur rails (22, 24) monté dans l'unité porteuse (12), sur lequel le corps de support (14) peut être déplacé en translation.
